# EUROPEAN PATENT SPECIFICATION

(11) **EP 4 379 034 B1**
(45) Date of publication and mention of the grant of the patent: **15.10.2025**
(21) Application number: 22210217.0
(22) Date of filing: 29.11.2022
(51) Int. Cl.: C12M 1/00, C13K 1/06, C12N 1/10, C12N 1/20, C12N 5/00

(54) **METHODS FOR THE PREPARATION OF CULTURE MEDIA BASED ON SACCHARIFIED PLANT EXTRACTS AND RELATED PRODUCTS**
VERFAHREN ZUR HERSTELLUNG VON KULTURMEDIEN AUF BASIS VON SACCHARIFIZIERTEN PFLANZENEXTRAKTEN UND VERWANDTEN PRODUKTEN
PROCÉDÉS DE PRÉPARATION DE MILIEUX DE CULTURE À BASE D'EXTRAITS DE PLANTES SACCHARIFIÉS ET PRODUITS ASSOCIÉS

(43) Date of publication of application: 05.06.2024
(73) Proprietor: ZHAW - Zürcher Hochschule für Angewandte Wissenschaften, 8820 Wädenswil (CH)
(72) Inventor: Eibl, Regine, Wädenswil (CH); Eibl, Dieter, Wädenswil (CH); Hühn, Tilo, Wädenswil (CH)
(74) Representative: Grünecker Patent- und Rechtsanwälte PartG mbB

(56) References cited:
- EP-A1- 3 556 222
- WO-A1-2014/100685
- DE-A1- 102004 026 152
- JP-A- 2008 000 100
- US-A1- 2021 284 700

## Description

### FIELD OF INVENTION

This invention relates to methods and/or techniques for a cost-effective and sustainable preparation of complex cell culture media or fermentation media on the basis of saccharified starch-containing plants.

### BACKGROUND OF THE INVENTION

Culture media are a source of nutrients and growth factors required for the growth of microorganisms and cells *ex-vivo* under laboratory conditions.

Many types of culture media, which may be classified based on their nutrient composition, consistency or their use in life science laboratories, have been developed to grow selective or desired microorganisms, cells or small plants.

Typically, a general distinction is being made between defined and undefined media.

A defined medium (also known as chemically defined medium or synthetic medium) is a chemically characterised medium produced from pure chemical substances, wherein no yeast, animal, or plant tissue is present. As long as their composition is defined, such media may also be prepared by using purified plant extracts, such as plant proteins (see WO 2021/148 955 A1) or plant peptides and lipids (see WO 98/15614).

On the other hand, an undefined medium (i.e. a basal or complex medium) is characterised in that it contains a carbon source, water, various salts a source of amino acids and nitrogen in unknown quantities. As an example, the addition of organic supplements from natural origin (i.e. coconut water, yeast extract, malt extract, potato extract, banana homogenisate algal compounds) to prepare undefined plant culture media is reviewed in the publication Z. Molnár et al, Acta Biologica Szegediensis 2011, 55(1), 123-127. US 2011/0212489 A1 discloses a culture medium which comprises a hydrolysate of plant seeds containing protein and oil (including sunflower seeds). US 2004/0185561 A1 discloses a cell culture medium which comprises a non-hydrolysed plant extract (e.g. from nuts, peas, or potatoes) and is tested for its growth-promoting effect as part of a cultivation of animal cells, especially keratinocytes. Further examples of culture media incorporating plant extracts are disclosed in US 2021/284700 A1 and WO 2014/100685 A1.

In commercial cell culture technology, culture media play a very important role in the efficiency and economic viability of production processes at industrial scale. Recent publications suggest that plant cell cultures or their extracts may be used as foodstuffs (see, e.g., E. Nordlund et al., Food Res Int. 2018, 107, 297-305 and R. Eibl et al., Appl. Microbiol. Biotechnol. 2018, 102, 8661-8675) and be manufactured with less energy and lower possible impact on the environment compared to whole plants, and independently of location and season. However, the use of defined cell culture media requires elaborate purification protocols and/or chemicals with a high degree of purity, which is a major cost driver in the transfer of such processes to a commercially relevant scale. While the addition of organic supplements from natural sources (e.g. to prepare undefined plant culture media) may advantageously influence cell survival, adhesion and proliferation, it still remains desirable to effectively produce culture media which contain a macronutrients, micronutrients, vitamins, amino acids or nitrogen supplements, source(s) of carbon and growth regulators at reduced processing costs.

Moreover, taking into account that common culture media used in the food science sector are usually derived from pharmaceutical science with a different set of legal requirements for approval, a direct transfer of such technologies to commercial food science for the preparation of cell culture-based food is a major challenge (due to the necessary declaration as novel food, for example). For instance, the addition of hormone supplements for control of cell differentiation may be subject to declaration depending on the application and region.

Hence, it would be desirable to provide culture media for food applications which may be produced sustainably and inexpensively, and which do not solely rely upon chemical *in-vitro* synthesis of such hormone additives.

DE 102004026152 A1 discloses a process for the biosynthesis of chemicals which involves liquefaction and saccharification of starch-containing plants to provide a carbon source for precision fermentation. Further examples of saccharidified materials derived from starch-containing plants are disclosed in EP 3 556 222 A1 and JP 2008-000100 A. However, starch liquefaction and saccharification processes typically involve harsh conditions, i.e. high temperatures (well above the gelling point of starch) at which thermolabile constituents of the starch-containing plant are decomposed. Therefore, it remains desirable to provide a process for the preparation of culture media, which preserves the heat-sensitive components (e.g. antioxidants and vitamins) of the plant, making the thus produced media useful for a wider range of applications.

### SUMMARY OF THE INVENTION

The present invention solves this object with the subject matter of the claims as defined herein. The advantages of the present invention will be further explained in detail in the section below and further advantages will become apparent to the skilled artisan upon consideration of the invention disclosure.

Generally speaking, in one aspect the present invention provides a method for the preparation of a cell culture medium or fermentation medium, comprising: a) providing a starch-containing plant; b) grinding the starch-containing plant to a mean particle size of 500 µm or less to provide a mash; c) subjecting the ground mash to a phase separation resulting in a solid phase and at least one liquid phase; d) separately processing the solid phase and the at least one liquid phase; e) recombining the separately processed phases; and f1) adding water to the recombined phases to provide the cell culture medium or fermentation medium; wherein in step d), the separated solid phase is subjected to a saccharification step; and wherein during steps a) to e), the at least one liquid phase is not subjected to temperatures of 55°C or above.

In a second aspect, the present invention relates to a method for the preparation of a supplement for a cell culture medium or fermentation medium, comprising: a) providing a starch-containing plant; b) grinding the starch-containing plant to a mean particle size of 500 µm or less to provide a mash; c) subjecting the ground mash to a phase separation resulting in a solid phase and at least one liquid phase; d) separately processing the solid phase and the at least one liquid phase; e) recombining the separately processed phases; and f2) subjecting the at least one liquid phase or the recombined phases to a drying and/or concentration step to provide the supplement for a cell culture medium or fermentation medium; wherein in step d), the separated solid phase is subjected to a saccharification step; and wherein during steps a) to e), the at least one liquid phase is not subjected to temperatures of 55°C or above.

### BRIEF DESCRIPTION OF THE DRAWINGS

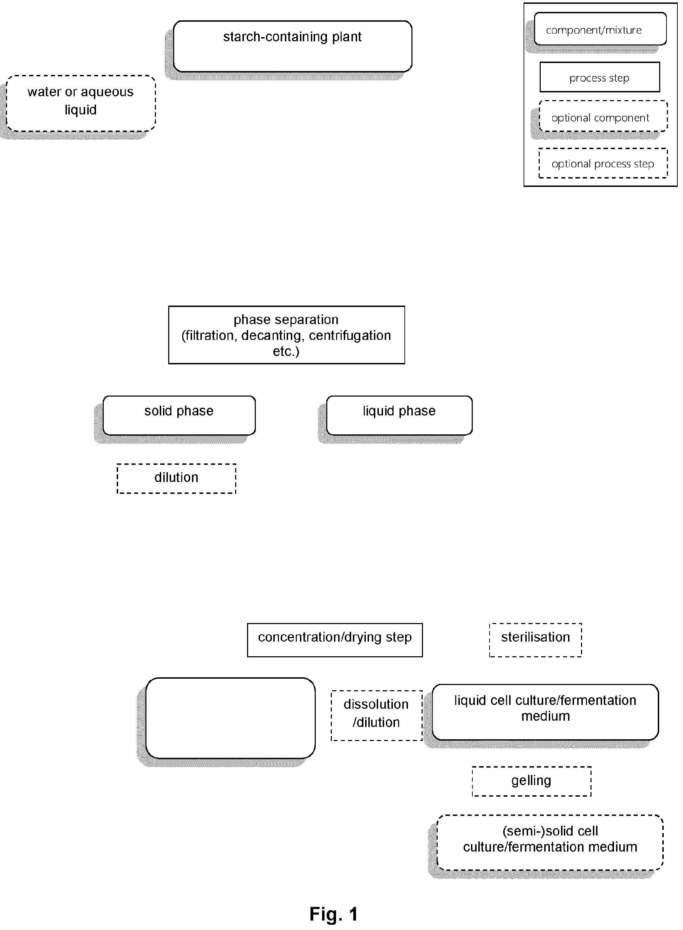

FIG. 1 is a flow chart illustrating examples of the methods according to the first and second embodiments of the present invention.

### DETAILED DESCRIPTION OF THE INVENTION

For a more complete understanding of the present invention, reference is now made to the following description of the illustrative embodiments thereof:

### Methods for the Preparation of Cell Culture or Fermentation Media

In a first embodiment, the present invention generally relates to a method for the preparation of a cell culture medium or fermentation medium, comprising: a) providing a starch-containing plant; b) grinding the starch-containing plant to a mean particle size of 500 µm or less to provide a mash; c) subjecting the ground mash to a phase separation resulting in a solid phase and at least one liquid phase; d) separately processing the solid phase and the at least one liquid phase; e) recombining the separately processed phases; and f1) adding water to the recombined phases to provide the cell culture medium or fermentation medium; wherein in step d), the separated solid phase is subjected to a saccharification step; and wherein during steps a) to e), the at least one liquid phase is not subjected to temperatures of 55°C or above.

In a second embodiment, the present invention relates to a method for the preparation of a supplement for a cell culture medium or fermentation medium, comprising: a) providing a starch-containing plant; b) grinding the starch-containing plant to a mean particle size of 500 µm or less to provide a mash; c) subjecting the ground mash to a phase separation resulting in a solid phase and at least one liquid phase; d) separately processing the solid phase and the at least one liquid phase; e) recombining the separately processed phases; and f2) subjecting the at least one liquid phase or the recombined phases to a drying and/or concentration step to provide the supplement for a cell culture medium or fermentation medium; wherein in step d), the separated solid phase is subjected to a saccharification step; and wherein during steps a) to e), the at least one liquid phase is not subjected to temperatures of 55°C or above.

Fig. 1 is a flow chart exemplifying a method for the preparation of a cell culture medium or fermentation medium (according to the first embodiment) and a method preparation of a supplement for a cell culture medium or fermentation medium (according to the second embodiment) in accordance with the present invention.

It will be understood that the the preferred features specified below with respect to the methods of the invention apply both for the method of the first and second embodiments, except for features that are mutually exclusive.

The terms "culture" or "cell culture", as used herein, refer to the maintenance, growth and/or differentiation of cells in an *in vitro*-environment.

"Fermentation" as used herein, includes traditional microbial fermentation, where microorganisms convert substrate into edible products, as well as precision fermentation, which involves the production of specific enzymes or protein ingredients, for example, by microorganisms.

Starch-containing plants for use in the method of the present invention are preferably selected from crop plants, preferably from cereals, legumes, oil crop, sugarcane, sugar beet, root vegetables, fruits and mixtures thereof, more preferably from corn, wheat, legumes, sugarcane, sugar beet, carrots, potatoes, sweet potatoes, fruits and mixtures thereof in view of favorable contents of carbon sources and their precursors for use in culture or fermentation media. In this respect, it may be preferred that the plant has a native starch content of at least 20 % by weight based on total dry weight of the plant.

Notably, one or more types of plants may be used for processing in the present invention. In some embodiments, it may be preferred that the starch-containing plant provided in step a) is present in an immature state or that the starch-containing plant is combined with one or more plants in an immature state (e.g. a microgreen) before being ground in step b) in order to make use of relatively high phytohormone contents. By introducing plant growth hormones accordingly, legal requirements for approval are facilitated, since no additional chemically produced or refined hormone supplements are required.

Before the grinding step b), the starch-containing plant is preferably washed and/or surface-sterilised. The washing and/or surface sterilisation steps are not particularly limited as long as they effectively reduce microbial surface contamination, and suitable methods may include chemical sterilisation (e.g., with ethanol solution (≥ 70% (v/v), 8-hydroxyquinoline and its derivatives or salts thereof, and sodium hypochlorite solution (e.g. 5% (v/v)) or the like), washing in sterile water, surface sterilisation or pasteurisation by means of heat and/or irradiation with ionizing rays (e.g. UV irradiation), ultrasound-based sterilisation and combinations thereof. In preferred embodiments, surface sterilisation is brought about by saturated steam pasteurisation in partial vacuum.

In step b), the starch-containing plant is ground to an average particle size of 500 µm or less. Average particle sizes of 200 µm or less, 100 µm or less, 90 µm or less, 50 µm or less or even 30 µm or less are especially preferred to enhance the release of nutrients into the liquid phase. The grinding of the mixture may be carried out in one or in multiple steps. The mill used for this purpose is not particularly limited and may include a pin mill, a hammer mill, a screen mill, a colloid mill or combinations thereof (e.g. in case of multiple grinding steps). The use of mills operating by use of vibration, oscillation and/or rotation at ultrasonic frequencies (such as e.g. a tooth colloid mill) in least one grinding (sub-)step may be preferable as it advantageously affects the extraction efficiency, presumably due to sonochemically induced reaction dynamics. The average particle size is measured as volume moment mean (*D*[4,3]), and is typically defined by all particle sizes contributing according to their volume fraction in the collective, so that the average particle size in the interval is weighted with the corresponding volume portion and all these weighted values are averaged arithmetically. The particle sizes and their distribution may be suitably determined by methods known in the art (e.g. by a particle size analyser).

In a preferred embodiment, the grinding step b) is carried out at a temperature lower than 40°C and preferably at temperatures of 0 to 35°C, such as e.g. lower or equal to ambient temperature (i.e. 25°C). In another preferred embodiment, the starch-containing plant may be subjected to wet grinding, more preferably in the presence of water, which may be optionally cooled before being added so as to further control the temperature regime. Such methods enable a high release of valuable plant constituents into the liquid phase while avoiding degradation of thermally sensitive antioxidants, vitamins and other biologically active agents.

In step c), the finely ground mash is subjected to a phase separation resulting in a solid phase and a liquid phase. The method of separating the phases is not particularly limited and may, for example, include filtration, decanting, centrifugation, or the like.

In step d), the solid phase is processed independently from the liquid phase and recombined with the liquid phase in step e).

As an example, independent processing of the solid phase may include a step of filtering, centrifuging or heating (e.g. under vacuum) the initially separated solid phase to further remove remaining liquids, and to recombine said liquid phase with the water phase from the initial separation step or at a later processing stage of said phases. Also, the liquid phase may be subjected to further purification steps, e.g. by membrane processes or filtration using vacuum rotation filters in order to remove fine particles.

According to the present invention, the solid phase is independently processed by subjecting the same to at least a saccharification step, wherein carbohydrate-containing materials (e.g., starchy materials, cellulosic materials, lignocellulosic materials) are converted to low molecular weight sugars, such as sucrose and/or glucose, for example. Starch is made from α-D(+)-glucose units and depending on the type of glucosidic bonds one distinguishes between amylose and amylopectin as the high-molecular building blocks of the starch grain.

In principle, all types of starch are suitable for saccharification. Native starch granules tend to be insensitive to acids or enzymes at room temperature. However, reactivity of starch increases upon heating in aqueous suspensions, which has the effect that the crystalline structure of starch is decomposed under water absorption, resulting in a gelatinisation. For this purpose, the starch-containing suspension is typically heated to temperatures of at least 70°C (usually between 90 to 170°C) for prolonged periods of time (at least 60 minutes, typically between 2-3 hours), sometimes under elevated pressure. In order to compensate for the viscosity increase during such a process, the starch concentration may be kept low (by prior or step-wise dilution of the suspension of the solid phase) or the starch may be preferably simultaneously hydrolysed in a liquefaction step, e.g. by addition of acids (e.g. to a pH of 1 to 4) and/or enzymes, prior to saccharification. Examples of starch-hydrolysing enzymes include, but are not limited to α-amylase, amyloglucosidase (alternatively referred to as glucoamylase) and variants thereof. Alternatively, the hydrolysis of starch may be realised by high-pressure steaming methods (e.g. in a Henze steamer), wherein temperatures of between 150 and 160° C and pressures of from 5 to 6 bar are being applied. However, in view of comparatively high energy costs, such methods are less preferred. Low-temperature methods, as the so-called cold mashing process, for example, are also known and are carried out at ambient pressure at temperatures lower than 65° C, but are applicable only for amylase-rich substrates and tend to result in substantially lower hydrolysis degrees.

In the saccharification step, which may be performed in a single or in multiple steps, the carbohydrate composition is adjusted. In a preferred embodiment, the saccharification step includes an enzymatic treatment, acidic treatment, solid catalyst-assisted microwave irradiation, thermal treatment under pressure, steam treatment or combinations thereof.

The thus obtained saccharified product may be purified, e.g., by removing suspended solids through filtration.

Notably, the separation of the liquid phase from the solid phase in step c) has the advantage that the liquid phase remains unaffected by the processing steps applied to the separated solid phase. Accordingly, the liquid phase does not need to be subjected to harsh processing conditions which inevitably apply during hydrolysis and saccharfication, so that heat-sensitive micro- and macronutrients transferred to the liquid phase during grinding and mixing may be preserved.

In this respect, it is preferred that during the entire preparation process, the liquid phase is not subjected to temperatures of 55°C or above. In further preferred embodiments, the liquid phase is not subjected to temperatures of 45°C or above, and especially preferably, the liquid phase is not subjected to temperatures of 35°C or above. On the other hand, the solid phase may be subjected to a thermal treatment at 65°C or above, such as 85°C or higher, or even 90°C or higher (e.g. during the liquefaction and/or saccharification step).

While not being necessary *per se*, further additives may be added to the mash or the liquid phase during or after step c) in order to further adjust, enrich and/or complement the nutrient profile according to the desired application. While not being limited thereto, such additives may comprise macronutrients (e.g., sources of nitrogen (N), phosphorus (P), potassium (K), calcium (Ca), magnesium (Mg) and sulphur (S)), micronutrients (e.g. sources of iron (Fe), manganese (Mn), zinc (Zn), boron (B), copper (Cu) and molybdenum (Mo)), source(s) of carbon (e.g., sucrose, glucose, sugar cane molasses, banana extract and coconut water), vitamins (e.g., thiamin (B1), nicotinic acid, pyridoxine (B6), biotin, folic acid, ascorbic acid, pantothenic acid, tocopherol (vitamin E), riboflavin, p-amino-and benzoic acid), amino acids or nitrogen supplements (e.g., casein hydrolysate, L-glutamine, L-asparagine, adenine, glycine, glutamine, asparagine, L-arginine, cysteine and L-tyrosine), undefined organic supplements (e.g., protein hydrolysates, coconut milk, yeast extract, malt extract, ground banana, orange juice and tomato juice), growth regulators (e.g. natural and synthetic auxins, cytokinins, gibberellins, abscisic acid and derivatives thereof) and solidifying agents, for example). Preferred additives include, but are not limited to: one or more salts including Ca²⁺, SO₄²⁻, Mg²⁺, PO₄³⁻, Cl⁻, NO₃⁻ and/or NH₄⁺ ions; one or more vitamins, preferably Vitamin B5; one or more growth regulators, preferably one or more selected from sterile coconut water, a cytokinin and an auxin, more preferably one or more selected from thidiazuron, zeatin or kinetin gibberellic acid, 2,4-dichlorophenoxyacetic acid (2,4-D), indoleacetic acid (IAA) or indolebutyric acid (IBA); and/or one or more phytohormones.

Once being processed according to the above description, upon prior recombination of the liquified saccharified extract obtained from the solid phase, the liquid phase may be diluted in water (preferably sterile water) in step f1), if necessary, to provide the cell culture or fermentation medium in ready-to-use form in step f).

If deemed necessary, the liquid phase may be sterilised. The method of sterilisation is not particularly limited and may include a heat treatment (e.g., autoclaving), irradiation with ionising rays, ultrasound-based sterilisation, sterile filtration through microporus filters and combinations thereof, while sterile filtration is preferably used in the presence of thermolabile constituents.

A solifidying agent (e.g., agar) may be added the liquid cell culture or fermentation medium at a concentration of 0.1 to 0.5 % wt.-% or 1 to 3.0 wt.-% to provide a semi-solid or solid medium, respectively.

Another convenient method for preparation of cell culture or fermentation media is to prepare a supplement in form of a dry powder or a concentrated stock solution, which can be be dissolved and/or diluted to the preferred concentration immediately before use, respectively. In this case, upon prior recombination of the liquified saccharified extract obtained from the solid phase, the liquid phase obtained in steps c) or after step d) is subjected to a concentration and/or drying step in step f2). Such a step may involve thermal treatment under low pressure conditions to expel water. However, low-temperature methods, such as freeze-drying, are preferred due to reduced loss of heat-sensitive nutrients.

### Media and Media Supplements

The present invention according to the first embodiment may be used to provide a cell culture medium or fermentation medium.

As outlined above, the cell culture medium or fermentation medium obtained according to the invention contains favorable concentrations of macronutrients, micronutrients, vitamins, amino acids or nitrogen supplements, source(s) of carbon and growth regulators, may be produced sustainably and inexpensively by regenerative or biological agriculture, and does not necessitate elaborate purification protocols in comparison to defined media.

The present invention according to second embodiment may be used to provide a supplement for a cell culture medium or fermentation medium.

Here, the supplement is being provided in powder form or as a concentrate, which may be stored in a refrigerator at 2- 4°C or in a freezer (-20°C), respectively. It will be understood that concentrated stock solutions and powders produced according to the aforementioned method need not to be used as constituent media upon dilution, but may also be employed as additive for known cell culture media or fermentation media.

## Claims

1. Method for the preparation of a cell culture medium or fermentation medium, comprising:
a) providing a starch-containing plant;
b) grinding the starch-containing plant to a volume moment mean (*D*[4,3]) particle size of 500 µm or less to provide a mash;
c) subjecting the ground mash to a phase separation resulting in a solid phase and at least one liquid phase;
d) separately processing the solid phase and the at least one liquid phase;
e) recombining the separately processed phases; and
f1) adding water to the recombined phases to provide the cell culture medium or fermentation medium;
wherein in step d), the separated solid phase is subjected to a saccharification step; and
wherein during steps a) to e), the at least one liquid phase is not subjected to temperatures of 55°C or above.

2. Method for the preparation of a supplement for a cell culture medium or fermentation medium, comprising:
a) providing a starch-containing plant;
b) grinding the starch-containing plant to a volume moment mean (D[4,3]) particle size of 500 µm or less to provide a mash;
c) subjecting the ground mash to a phase separation resulting in a solid phase and at least one liquid phase;
d) separately processing the solid phase and the at least one liquid phase;
e) recombining the separately processed phases; and
f2) subjecting the at least one liquid phase or the recombined phases to a drying and/or concentration step to provide the supplement for a cell culture medium or fermentation medium;
wherein in step d), the separated solid phase is subjected to a saccharification step; and
wherein during steps a) to e), the at least one liquid phase is not subjected to temperatures of 55°C or above.

3. Method according to any one of claims 1 or 2, wherein the plant is selected from crop plants, preferably from cereals, legumes, oil crop, sugarcane, sugar beet, root vegetables and fruits, more preferably from corn, wheat, legumes, sugarcane, sugar beet, carrots, potatoes, sweet potatoes and fruits.

4. Method according to any of claims 1 to 3, wherein the plant has a starch content of at least 20 % by weight based on total dry weight.

5. Method according to any of claims 1 to 4, wherein in step a) the starch-containing plant is present in an immature state.

6. Method according to any of claims 1 to 5, wherein the separately processed phases are subjected to a sterilisation step, independently or upon recombination of the phases, preferably by sterile filtration upon recombination of the phases.

7. Method according to any of claims 1 to 6, wherein the saccharification step includes an enzymatic treatment, acidic treatment, solid catalyst-assisted microwave irradiation, thermal treatment under pressure, steam treatment or combinations thereof.

8. Method according to any of claims 1 to 7, wherein the starch-containing plant is subjected to wet grinding in step b), preferably in the presence of water.

9. Method according to any of claims 1 to 8, wherein step b) is performed at temperatures below 40°C, more preferably at temperatures of 5 to 35°C.

10. Method according to any of claims 1 to 9, wherein in step b), the starch-containing plant is ground to a volume moment mean (*D*[4,3]) particle size of 90 µm or less, preferably 50 µm or less, more preferably 30 µm or less.

11. Method according to any of claims 1 to 10, wherein the solid phase is subjected to a liquefaction step prior to the saccharification step, preferably by slurrying the solid phase in water, gelatinising the slurry and hydrolysing the same in the presence of one or more types of starch hydrolysing enzymes.

12. Method according to any of claims 1 to 11, wherein during steps a) to e), the at least one liquid phase is not subjected to temperatures of 45°C or above.

13. Method according to any of claims 1 to 12, wherein the solid phase is subjected to a thermal treatment at 65°C or above, such as 85°C or higher.

14. Method according to any of claims 1 to 13, wherein one or more of the following is further added during or after step b):
one or more salts including Ca²⁺, SO₄²⁻, Mg²⁺, PO₄³⁻, Cl⁻, NO₃⁻ and/or NH₄⁺ ions;
one or more vitamins, preferably Vitamin B5;
one or more growth regulators, preferably one or more selected from sterile coconut water, a cytokinin and an auxin, more preferably one or more selected from thidiazuron, zeatin or kinetin gibberellic acid, 2,4-dichlorophenoxyacetic acid (2,4-D), indoleacetic acid (IAA) or indolebutyric acid (IBA); and/or
one or more phytohormones.

## Patentansprüche

1. Verfahren zur Herstellung eines Zellkulturmediums oder Fermentationsmediums, umfassend:
a) Bereitstellen einer stärkehaltigen Pflanze;
b) Mahlen der stärkehaltigen Pflanze auf eine mittlere Volumenmomentgröße (*D*[4,3]) der Partikel von 500 µm oder weniger, um eine Maische zu erhalten;
c) Unterziehen der gemahlenen Maische einer Phasentrennung, die zu einer festen Phase und mindestens einer flüssigen Phase führt;
d) getrenntes Verarbeiten der festen Phase und der mindestens einen flüssigen Phase;
e) Rekombinieren der separat verarbeiteten Phasen; und
f1) Hinzufügen von Wasser zu den wieder vereinigten Phasen, um das Zellkulturmedium oder Fermentationsmedium bereitzustellen;
wobei in Schritt d) die abgetrennte feste Phase einem Verzuckerungsschritt unterzogen wird; und
wobei während der Schritte a) bis e) die mindestens eine flüssige Phase keinen Temperaturen von 55 °C oder höher ausgesetzt wird.

2. Verfahren zur Herstellung eines Zusatzstoffes für ein Zellkulturmedium oder Fermentationsmedium, umfassend:
a) Bereitstellen einer stärkehaltigen Pflanze;
b) Mahlen der stärkehaltigen Pflanze auf eine mittlere Volumenmomentgröße (*D*[4,3]) von 500 µm oder weniger, um eine Maische zu erhalten;
c) Unterziehen der gemahlenen Maische einer Phasentrennung, die zu einer festen Phase und mindestens einer flüssigen Phase führt;
d) getrenntes Verarbeiten der festen Phase und der mindestens einen flüssigen Phase;
e) Rekombinieren der separat verarbeiteten Phasen; und
f2) Unterziehen der mindestens einen flüssigen Phase oder der wieder vereinigten Phasen einem Trocknungs- und/oder Konzentrationsschritt, um das Ergänzungsmittel für ein Zellkulturmedium oder Fermentationsmedium bereitzustellen;
wobei in Schritt d) die abgetrennte feste Phase einem Verzuckerungsschritt unterzogen wird; und
wobei während der Schritte a) bis e) die mindestens eine flüssige Phase keinen Temperaturen von 55 °C oder höher ausgesetzt wird.

3. Verfahren nach einem der Ansprüche 1 oder 2, wobei die Pflanze aus Kulturpflanzen ausgewählt ist, vorzugsweise aus Getreide, Hülsenfrüchten, Ölpflanzen, Zuckerrohr, Zuckerrüben, Wurzelgemüse und Früchten, noch bevorzugter aus Mais, Weizen, Hülsenfrüchten, Zuckerrohr, Zuckerrüben, Karotten, Kartoffeln, Süßkartoffeln und Früchten.

4. Verfahren nach einem der Ansprüche 1 bis 3, wobei die Pflanze einen Stärkegehalt von mindestens 20 Gew.-%, bezogen auf das gesamte Trockengewicht, aufweist.

5. Verfahren nach einem der Ansprüche 1 bis 4, wobei in Schritt a) die stärkehaltige Pflanze in einem unreifen Zustand vorliegt.

6. Verfahren nach einem der Ansprüche 1 bis 5, wobei die separat verarbeiteten Phasen einem Sterilisationsschritt unterzogen werden, unabhängig oder nach der Rekombination der Phasen, vorzugsweise durch sterile Filtration nach der Rekombination der Phasen.

7. Verfahren nach einem der Ansprüche 1 bis 6, wobei der Verzuckerungsschritt eine enzymatische Behandlung, eine Säurebehandlung, eine mit einem festen Katalysator unterstützte Mikrowellenbestrahlung, eine thermische Behandlung unter Druck, eine Dampfbehandlung oder Kombinationen davon umfasst.

8. Verfahren nach einem der Ansprüche 1 bis 7, wobei die stärkehaltige Pflanze in Schritt b) einer Nassvermahlung unterzogen wird, vorzugsweise in Gegenwart von Wasser.

9. Verfahren nach einem der Ansprüche 1 bis 8, wobei Schritt b) bei Temperaturen unter 40 °C, vorzugsweise bei Temperaturen von 5 bis 35 °C, durchgeführt wird.

10. Verfahren nach einem der Ansprüche 1 bis 9, wobei in Schritt b) die stärkehaltige Pflanze auf eine mittlere Volumenmomentgröße (*D*[4,3]) von 90 µm oder weniger, vorzugsweise 50 µm oder weniger, noch bevorzugter 30 µm oder weniger gemahlen wird.

11. Verfahren nach einem der Ansprüche 1 bis 10, wobei die feste Phase vor dem Verzuckerungsschritt einem Verflüssigungsschritt unterzogen wird, vorzugsweise durch Aufschlämmen der festen Phase in Wasser, Gelatinieren der Aufschlämmung und Hydrolysieren derselben in Gegenwart einer oder mehrerer Arten von stärkehydrolysierenden Enzymen.

12. Verfahren nach einem der Ansprüche 1 bis 11, wobei während der Schritte a) bis e) die mindestens eine flüssige Phase keinen Temperaturen von 45 °C oder darüber ausgesetzt wird.

13. Verfahren nach einem der Ansprüche 1 bis 12, wobei die feste Phase einer thermischen Behandlung bei 65 °C oder darüber, beispielsweise 85 °C oder höher, unterzogen wird.

14. Verfahren nach einem der Ansprüche 1 bis 13, wobei während oder nach Schritt b) zusätzlich eines oder mehrere der folgenden Mittel zugegeben werden:
ein oder mehrere Salze, die Ionen ausgewählt aus Ca²⁺, SO₄²⁻, Mg²⁺, PO₄³⁻, Cl⁻, NO₃⁻und/oder NH₄⁺ enthalten;
ein oder mehrere Vitamine, vorzugsweise Vitamin B5;
ein oder mehrere Wachstumsregulatoren, vorzugsweise ein oder mehrere, ausgewählt aus sterilem Kokosnusswasser, einem Cytokinin und einem Auxin, noch bevorzugter ein oder mehrere, ausgewählt aus Thidiazuron, Zeatin oder Kinetin, Gibberellinsäure, 2,4-Dichlorphenoxyessigsäure (2,4-D), Indolessigsäure (IAA) oder Indolbuttersäure (IBA); und/oder
ein oder mehrere Phytohormone.

## Revendications

1. Procédé de préparation d'un milieu de culture cellulaire ou d'un milieu de fermentation, comprenant :
a) la fourniture d'une plante contenant de l'amidon ;
b) le broyage de la plante contenant de l'amidon jusqu'à obtenir une taille de particule moyenne en volume (D[4,3]) inférieure ou égale à 500 µm pour obtenir une purée ;
c) la soumission de la purée broyée à une séparation de phases résultant en une phase solide et au moins une phase liquide ;
d) le traitement séparé de la phase solide et de ladite au moins une phase liquide ;
e) la recombinaison des phases traitées séparément ; et
f1) l'ajout d'eau aux phases recombinées pour fournir le milieu de culture cellulaire ou le milieu de fermentation ;
dans lequel, à l'étape d), la phase solide séparée est soumise à une étape de saccharification ; et
dans lequel, durant les étapes a) à e), ladite au moins une phase liquide n'est pas soumise à des températures supérieures ou égales à 55 °C.

2. Procédé de préparation d'un supplément pour un milieu de culture cellulaire ou un milieu de fermentation, comprenant :
a) la fourniture d'une plante contenant de l'amidon ;
b) le broyage de la plante contenant de l'amidon jusqu'à obtenir une taille de particule moyenne en volume (D[4,3]) inférieure ou égale à 500 µm pour obtenir une purée ;
c) la soumission de la purée broyée à une séparation de phases résultant en une phase solide et au moins une phase liquide ;
d) le traitement séparé de la phase solide et de ladite au moins une phase liquide ;
e) la recombinaison des phases traitées séparément ; et
f2) la soumission de ladite au moins une phase liquide ou des phases recombinées à une étape de séchage et/ou de concentration pour fournir le supplément pour un milieu de culture cellulaire ou un milieu de fermentation ;
dans lequel, à l'étape d), la phase solide séparée est soumise à une étape de saccharification ; et
dans lequel, durant les étapes a) à e), ladite au moins une phase liquide n'est pas soumise à des températures supérieures ou égales à 55 °C.

3. Procédé selon l'une quelconque des revendications 1 et 2, dans lequel la plante est sélectionnée parmi les plantes cultivées, de préférence parmi les céréales, les légumineuses, les oléagineux, la canne à sucre, la betterave sucrière, les légumes-racines et les fruits, de préférence encore parmi le maïs, le blé, les légumineuses, la canne à sucre, la betterave sucrière, les carottes, les pommes de terre, les patates douces et les fruits.

4. Procédé selon l'une quelconque des revendications 1 à 3, dans lequel la plante présente une teneur en amidon d'au moins 20 % en masse sur la base de la masse sèche totale.

5. Procédé selon l'une quelconque des revendications 1 à 4, dans lequel, à l'étape a), la plante contenant de l'amidon est présente dans un état immature.

6. Procédé selon l'une quelconque des revendications 1 à 5, dans lequel les phases traitées séparément sont soumises à une étape de stérilisation, indépendamment de la recombinaison des phases ou lors de celle-ci, de préférence par filtration stérile lors de la recombinaison des phases.

7. Procédé selon l'une quelconque des revendications 1 à 6, dans lequel l'étape de saccharification inclut un traitement enzymatique, un traitement acide, une irradiation par micro-ondes assistée par un catalyseur solide, un traitement thermique sous pression, un traitement à la vapeur, ou des combinaisons de ceux-ci.

8. Procédé selon l'une quelconque des revendications 1 à 7, dans lequel la plante contenant de l'amidon est soumise à un broyage humide à l'étape b), de préférence en présence d'eau.

9. Procédé selon l'une quelconque des revendications 1 à 8, dans lequel l'étape b) est réalisée à des températures inférieures à 40 °C, de préférence à des températures de 5 °C à 35 °C.

10. Procédé selon l'une quelconque des revendications 1 à 9, dans lequel, à l'étape b), la plante contenant de l'amidon est broyée jusqu'à obtenir une taille de particule moyenne en volume (D[4,3]) inférieure ou égale à 90 µm, de préférence inférieure ou égale à 50 µm, de préférence encore inférieure ou égale à 30 µm.

11. Procédé selon l'une quelconque des revendications 1 à 10, dans lequel la phase solide est soumise à une étape de liquéfaction avant l'étape de saccharification, de préférence en mettant en suspension la phase solide dans de l'eau, en gélatinisant la suspension, et en hydrolysant celle-ci en présence d'un ou plusieurs types d'enzymes d'hydrolyse de l'amidon.

12. Procédé selon l'une quelconque des revendications 1 à 11, dans lequel, durant les étapes a) à e), ladite au moins une phase liquide n'est pas soumise à des températures supérieures ou égale à 45 °C.

13. Procédé selon l'une quelconque des revendications 1 à 12, dans lequel la phase solide est soumise à un traitement thermique à une température supérieure ou égale à 65 °C, par exemple supérieure ou égale à 85 °C.

14. Procédé selon l'une quelconque des revendications 1 à 13, dans lequel un ou plusieurs des éléments suivants sont en outre ajoutés durant ou après l'étape b) :
un ou plusieurs sels incluant des ions Ca²⁺, SO₄²⁻, Mg²⁺, PO₄³⁻, Cl⁻, NO₃⁻ et/ou NH₄⁺ ;
une ou plusieurs vitamines, de préférence la vitamine B5 ;
un ou plusieurs régulateurs de croissance, de préférence un ou plusieurs éléments sélectionnés parmi l'eau de coco stérile, une cytokinine et une auxine, de préférence encore un ou plusieurs éléments sélectionnés parmi le thidiazuron, la zéatine ou la kinétine, l'acide gibbérellique, l'acide 2,4-dichlorophénoxyacétique (2,4-D), l'acide indoleacétique (IAA) ou l'acide indolebutyrique (IBA) ; et/ou
une ou plusieurs phytohormones.
